# EUROPEAN PATENT APPLICATION

(11) **EP 3 420 905 A1**
(43) Date of publication of application: **02.01.2019**
(21) Application number: 17305819.9
(22) Date of filing: 29.06.2017
(51) Int. Cl.: A61B 6/00, G06T 5/00

(54) **IMAGE CONTRAST ENHANCEMENT OF AN X-RAY IMAGE**

(71) Applicant: Koninklijke Philips N.V., 5656 AE Eindhoven (NL)
(72) Inventor: HOORNAERT, Bart Pierre Antoine Jozef, 5656 AE Eindhoven (NL); FLORENT, Raoul, 5656 AE Eindhoven (NL); LEVRIER, Claire, 5656 AE Eindhoven (NL); GEVERS, Bernardus Marius Hubertus, 5656 AE Eindhoven (NL)
(74) Representative: de Haan, Poul Erik

(57) **Abstract**

The present invention relates to image contrast enhancement. In order to provide improved and stable contrast enhancement for each acquired image, a device (10) for image contrast enhancement of an X-ray image of a vascular structure is provided. The device (10) comprises an input unit (12) and a processing unit (14). The input unit (12) is configured to provide a current acquisition parameter setting having at least one acquisition parameter, and to provide a generic vessel model. The processing unit is configured to assess a current patient build based on the current acquisition parameter setting, to assess at least one contrast parameter for at least a part of the main vessels of the generic vessel model based on the assessed current patient build and the current acquisition parameter setting and to determine an adaptive image contrast enhancement based on the at least one assessed contrast vessel parameter. The adaptive image contrast enhancement is applied to an X-ray image of a vascular structure, which is acquired with the current acquisition parameter setting, in order to generate a contrast enhanced X-ray image.

## Description

### FIELD OF THE INVENTION

The present invention relates to a device for image contrast enhancement of an X-ray image of a vascular structure, to a system with such a device, to a method for image contrast enhancement of an X-ray image of a vascular structure, to a computer program element for controlling such a device and to a computer readable medium having stored the program element.

### BACKGROUND OF THE INVENTION

In X-ray images, contrast enhancement (CE) is provided to improve image quality for the viewer. Contrast enhancement may be realized in the framework of frequency decomposition. For example, the image is decomposed into a certain number of frequency sub-bands, for instance, with a so-called Laplacian Pyramid. The anatomical or interventional details one is willing to enhance are spread across several of those frequency sub-bands. Gains, which may depend on the local intensity are then applied to the content of those sub-bands, before the total spectrum is re-composed, thus leading to an enhancement. In order to reduce noise, contrast, and motion artifacts in the images decomposed from a dual-energy imaging system, in US 2003/0152258 A1, a technique is provided for selecting parameters for the dual-energy decomposition process. However, it has been shown that contrast enhancement is not always leading to sufficient results.

### SUMMARY OF THE INVENTION

There may thus be a need to provide improved and stable contrast enhancement for each acquired image.

The object of the present invention is solved by the subject-matter of the independent claims; further embodiments are incorporated in the dependent claims. It should be noted that the following described aspects of the invention apply also for the device for image contrast enhancement of an X-ray image of a vascular structure, for the system with such a device, for the method for image contrast enhancement of an X-ray image of a vascular structure, and for the computer program element for controlling such a device and also for the computer readable medium having stored the program element.

According to a first aspect, a device is provided for image contrast enhancement of an X-ray image of a vascular structure. The device comprises an input unit and a processing unit. The input unit is configured to provide a current acquisition parameter setting having at least one acquisition parameter, and to provide a generic vessel model. The processing unit is configured to assess a current patient build based on the current acquisition parameter setting, to assess at least one contrast parameter for at least a part of the main vessels of the generic vessel model based on the assessed current patient build and the current acquisition parameter setting and to determine an adaptive image contrast enhancement based on the at least one assessed contrast vessel parameter. The adaptive image contrast enhancement is applied to an X-ray image of a vascular structure, which is acquired with the current acquisition parameter setting, in order to generate a contrast enhanced X-ray image.

In an example, the current acquisition parameter setting is read-in in the processing unit.

In an example, the generic vascular structure is read-in in the processing unit.

In an example, the generic vascular model is provided at least for the targeted vascular structure for which an image is to be captured.

The term "acquired" can also be referred to as captured.

The term "assessing" relates to a determination of the current patient build, which determination may be a result of an estimating or assessing procedure. For example, the assessment is provided as an estimation.

The term "generic vessel model" can also be referred to as a theoretical model of the human's vessels.

The term "generic patient model" relates to a model of a subject, i.e. of a patient. For example, the generic patient model relates to a theoretical structure of a patient. For example, the generic patient model is based on empiric data, such as from a large number of actual patients that have been examined over time. In another example, the generic patient model is based on an ideal setup of an ideal patient. The generic patient model may be provided as a simplified or ideal patient model.

The "current patient build" relates to a built or constitution of the current patient, i.e. a state of the current patient. The current patient build or patient built can also be referred to as estimated patient anatomy.

In an example, the generic vessel model is based on empiric data, such as from a large number of actual vessel structures that have been acquired over time. In another example, the generic vessel model is based on an ideal setup of an ideal patient. The generic vessel model may be provided as a simplified or ideal vascular structure.

In an example, the assessed patient build is deduced from a previous radiation, i.e. that means that the assessed patient build is estimated based on the applied exposure parameters, such as the kV and the filter thickness. In an example, the filter is a Cu-Filter.

The term "at least one contrast parameter" relates to the contrast in a respective image. This contrast is predicted, i.e. assessed or estimated. The at least one contrast parameter may comprise one or more parameters that are characteristic of the respective predicted contrast. The assessed at least one contrast parameter relates to an assessed contrast that is expected when acquiring a patient having the assessed current patient build and when acquiring with the current acquisition parameter setting.

The term "adaptive" is related to a variable or dynamic adaptation of the contrast enhancement depending on the patient and the acquisition parameter settings.

The adaptive image contrast enhancement can be referred to boosting or tuning. It indicates how the image has to be modified with regards to contrast of the image. The adaptive image contrast enhancement is a dynamic boosting of images, e.g. the applied contrast enhancement procedure is determined by the current acquisition parameter setting, since the current situation is taken into account.

The adapted image contrast enhancement could also be referred to as a contrast value for improving the image quality. The adapted image contrast enhancement can also be referred to as predicted contrast enhancement, or predicted adapted contrast enhancement.

In an example, the processing unit is also configured to determine if the assessed contrast parameter is below, equal or above a predetermined threshold.

In an example, the processing unit performs an algorithm for determining adapted image contrast enhancement for the generic vessel model based on the assessed vessel contrast parameter.

In an example, the adapted contrast image enhancement can be applied by a contrast enhancement procedure or program operating on the processing unit.

In an example, the input unit, or input interface, reads-in the current acquisition parameter setting.

In an example, the acquisition parameters allow to estimate the build of the patient. In an example, the input unit is configured to provide a generic patient model. In an example, a generic patient model comprises the generic vessel model.

In an example, the processing unit is configured to assess a current patient build by modifying the generic patient model based on the current acquisition parameter setting.

In an example, the device is also used for examinations of the gastro-intestinal tract.

In the case of heavily-built patients, for example, some of the exposure parameters (like kV) have to be increased in order to maintain acceptable image quality (in terms of noise level). This leads to images with very poor contrasts, which would need a high level of boosting. On the contrary, acquisitions of thinner patients for which image quality is usually far better could suffer from the same level of boosting, which could entail saturation, or vessel-over-vessel visibility loss. In an example, when the exposure parameters are preferably reduced, e.g. because of patient's disease, a respective adapted acquisition parameter setting is applied. This assessed contrast vessel parameter for the generic vessel model is to be predicted to be below or at least different of another value, which can be defined by the determination of the assessed contrast vessel parameter. Based on this information, one can deduce the contrast of vessels to be captured by X-ray prior to vessel boosting or contrast enhancement.

Hence, the boosting adapts the contrast of the image based on the current acquisition parameters, which leads to images with improved contrast. With regards to the above-mentioned example of a thinner patient, the kV value needs to be lower compared to other patients. The boosting of the to be captured image of the vessels of the patient can be adapted by considering this adaptation of this acquisition parameter.

Therefore, the adapted contrast enhancement relies on information to improve the image quality based on the current injection setting. In other words, the adapted image contrast enhancement is a dynamic boosting of images, e.g. the applied contrast enhancement procedure is determined by the current acquisition parameter settings which is in turn depending on the patient specifics. With regards to the above-mentioned example of a thinner patient, the exposure parameter (e.g. kV value) may need to be lowered compared to thicker patients. The boosting of the to be captured image of the vessels of the patient can be adapted by considering this adaptation of this acquisition parameter.

In an example, the adapted image contrast enhancement for the generic vessel model based on the assessed contrast vessel parameter is performed by an enhancement algorithm. For example, the assessed contrast distribution of the main vessels is used to scale look-up-tables and gains that are applied on the content of the relevant frequency sub-bands of the current X-ray image. In an example, for a typical patient at a typical geometry, the vessel contrast in the image, obtained with a typical acquisition parameter, can be postulated to be the desired or reference contrast, or the so-called threshold. It would have a reference value for a contrast enhancement parameter. In other situations, the vessel contrast in the image will be less (for example: half the contrast). Hence the vessels are not only less visible, also the interventionalist or physician misses an absolute reference level. The predicted contrast enhancement would have a specific value. Hence the vessel contrast in the image could be boosted with this specific value.

As a result, improved and stable contrast enhancement applied X-ray images can be achieved, e.g. optimized opacification and delineation in the anatomy, including lesions, can be achieved.

According to an example, the device further comprises an output unit. The processing unit is configured to apply the adaptive image contrast enhancement to the acquired X-ray image of the vascular structure and to generate a dynamically contrast enhanced X-ray image. The output unit is configured to provide the contrast enhanced X-ray image. Preferably, the output unit is a display unit.

The output unit can also be referred to as an output interface, such as a graphical user output interface. In an example, the output unit transmits modified image data after applying the adapted image enhancement on the to be captured image.

According to a second aspect, an X-ray imaging system is provided. The X-ray imaging system comprises an X-ray imaging arrangement, a device for image contrast enhancement of an X-ray image of a vascular structure. The X-ray imaging arrangement is configured to acquire the X-ray image of a vascular structure with the contrast acquisition parameter setting. The X-ray imaging arrangement is configured for variation and/or adaptation of the current acquisition parameter setting.

In an example, the data transmission can be a wireless connection to the processing unit or other type of data transmission, e.g. via cable connection.

According to an example, the current acquisition parameter setting comprises: an exposure parameter setting of at least one of the group of kV-value and filter; and/or a geometry parameter setting of at least one of the group of gantry geometry parameter and detector geometry parameter.

In an example, the current acquisition parameter setting comprises a contrast injection setting comprising a contrast injection parameter of at least one of the group of: delivered iodine concentration, injection timing, injection volume, injection speed, viscosity of iodine concentration, and injection real-time pressure curve. The term "real-time pressure curve" relates to real-time concentration of iodine injected, i.e. the pressure with which the concentration is injected.

According to a third aspect, a method is provided for image contrast enhancement of an X-ray image of the vascular structure. The method comprises the following steps.
a) In a first step, a current acquisition parameter setting having at least one acquisition parameter is provided.
b) In a second step, a current patient build is assessed based on the current acquisition parameter setting.
c) In a third step, a generic vessel model is provided;
d) In a fourth step, at least one contrast parameter for at least a part of the main vessels of the generic vessel model is assessed based on the assessed current patient build and the current acquisition parameter setting.
e) In a fifth step, an adaptive image contrast enhancement based on the at least one assessed contrast vessel parameter is determined. The adaptive image contrast enhancement is applied to an X-ray image of a vascular structure, which is acquired with the current acquisition parameter setting, in order to generate a contrast enhanced X-ray image.

In an example, the current patient build is assessed by modifying the generic patient model based on the acquisition parameter.

In an example, the acquisition parameters allow to estimate the build of the patient.

In an example, in step d), the assessing of the contrast parameter is performed at least for a targeted vascular structure for which the image of the patient's vascular structure is captured.

In an example, before step e), a current parameter of the acquired X-ray image is determined; and wherein step e) is performed if the current built parameter is below a predetermined threshold.

In an example, steps a) to e) are arranged in a different order. For example, the generic patient model and the current acquisition parameter as well as the generic vessel model are provided as first step. In any case, step c) is provided before step d). Step c) can hence also be provided as a further sub-step in step a).

According to an aspect, it is provided to enhance the image quality of an acquired X-ray image by adapting contrast boosting or contrast enhancement depending on the circumstances of the acquired image. As a result, the contrast enhancement is done dynamically. For example, when an image is captured for a thicker patient, the adapted contrast enhancement parameter is adapted to a higher level than for a thinner patient.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of a device for image contrast enhancement of an X-ray image of a vascular structure;
Fig. 2 shows an example of an X-ray imaging system with such a device;
Fig. 3 illustrates an example of a method for image contrast enhancement of an X-ray image of a vascular structure; and
Fig. 4 illustrates another example of such a method.

### DETAILED DESCRIPTION OF EMBODIMENTS

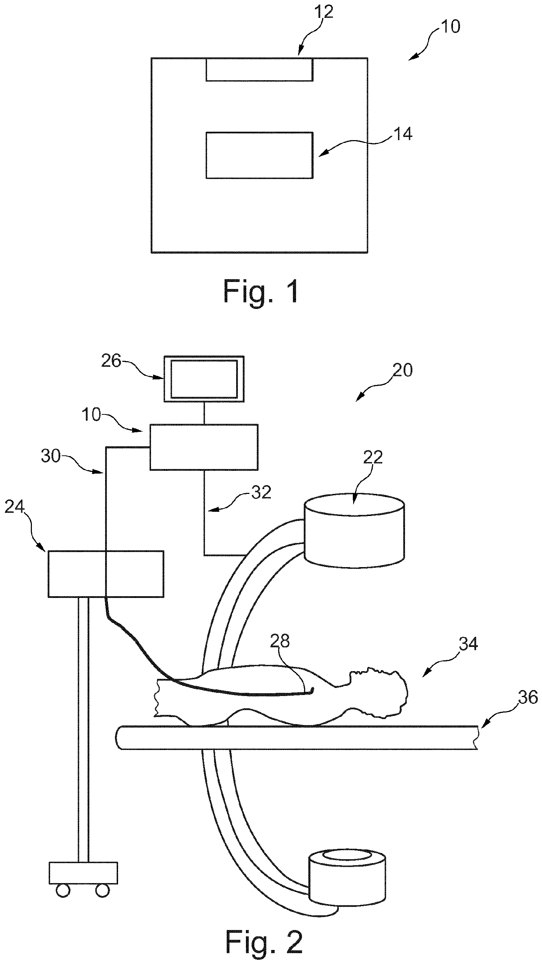

Fig. 1 shows a device 10 for image contrast enhancement of an X-ray image of a vascular structure. The device 10 comprises an input unit 12 and a processing unit 14. The input unit 12 is configured to provide a current acquisition parameter setting having at least one acquisition parameter, and to provide a generic vessel model. The processing unit 14 is configured to assess a current patient build based on the current acquisition parameter setting, to assess at least one contrast parameter for at least a part of the main vessels of the generic vessel model based on the assessed current patient build and the current acquisition parameter setting and to determine an adaptive image contrast enhancement based on the at least one assessed contrast vessel parameter. The adaptive image contrast enhancement is applied to an X-ray image of a vascular structure, which is acquired with the current acquisition parameter setting, in order to generate a contrast enhanced X-ray image.

In an example, an output unit 26 (as shown in Fig. 2) is provided. In an example, the processing unit 14 is configured to apply the adaptive image contrast enhancement to the acquired X-ray image of the vascular structure and to generate a dynamically contrast enhanced X-ray image. The output unit 26 is configured to provide the contrast enhanced X-ray image. Preferably, the output unit 26 is a display unit.

Fig. 2 shows an X-ray imaging system 20. The X-ray imaging system 20 comprises an X-ray imaging arrangement 22 and an example of the device 10 for image contrast enhancement of an X-ray image of a vascular structure. The X-ray imaging arrangement 22 is configured to acquire the X-ray image of a vascular structure with the contrast acquisition parameter setting. The X-ray imaging arrangement is configured for variation and/or adaptation of the current acquisition parameter setting.

In an example, the acquisition parameter setting is transmitted to the device 10 (shown by connection line).

In an example, the acquired X-ray image is transmitted to the device 10, as shown by connection line 32.

Fig. 2 further shows a patient 34 lying on a table 36 during the acquisition of the X-ray image.

In an example, the current acquisition parameter setting is transmitted to the device in parallel to the acquisition of the X-ray image.

In another example, the current, i.e. applied contrast acquisition parameter setting is transmitted after acquisition of the X-ray image.

In an example, the current acquisition parameter setting comprises an exposure parameter setting of at least one of the group of kV-value and filter; and/or a geometry parameter setting at least one of the group of gantry geometry parameter and detector geometry parameter.

In an example, the current acquisition parameter setting comprises an absorber parameter setting at least one of the group of: wedge filters, bowtie filters, neck filter, leg filters.

In an optionally shown example, a contrast injection arrangement 24 is adapted to perform a contrast injection 28 with a current contrast injection setting having at least one contrast injection parameter.

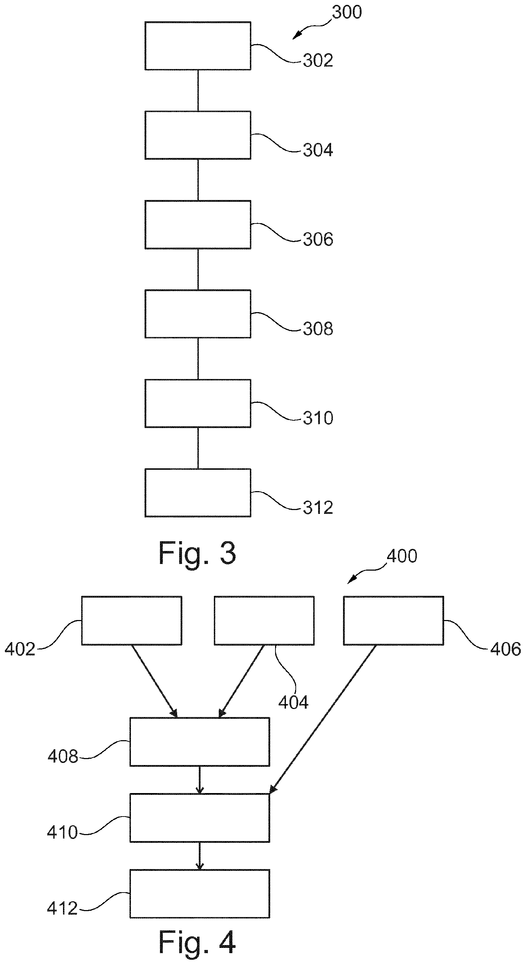

Fig. 3 shows a method 300 for image contrast enhancement of an X-ray image of the vascular structure. The method comprises the following steps. In a first step 302, also referred to step as step a), a current acquisition parameter setting having at least one acquisition parameter is provided. In a next step 304, also referred to as step b), a current patient build based on the current acquisition parameter setting is assessed. In a further next step 306, also referred to as step c), a generic vessel model is provided. In a next step 308, also referred to as step d), at least one contrast parameter for at least a part of the main vessels of the generic vessel model based on the assessed current patient build and the current acquisition parameter setting is assessed. In a still next step 310, also referred to as step e), an adaptive image contrast enhancement based on the at least one assessed contrast vessel parameter is determined. The adaptive image contrast enhancement is applied to an X-ray image of a vascular structure, which is acquired with the current acquisition parameter setting, in order to generate a contrast enhanced X-ray image.

In an example, the current acquisition parameter setting comprises an exposure parameter setting of at least one of the group of: kV value and filter.

The kV value relates to the voltage or electrical potential applied to the X-ray tube.

The filter relates to an anode surface where low-energy photons are absorbed or filtered out as they attempt to pass the anode surface. The amount of filtration is generally dependent on the composition and thickness of material through which the X-ray beam passes and is generally what determines the shape of the low-energy end of the spectrum curve.

In an example, the filter is a Cu-filter. In an example, the thickness of the Cu filter is adaptable. In an example, the current acquisition parameter setting comprises a geometry parameter setting at least one of the group of: gantry geometry parameter and detector geometry parameter. The geometry setting includes gantry geometrical settings and detector geometrical settings, such as magnification and format selection.

In an optional example, the current acquisition parameter setting comprises a contrast injection setting comprising a contrast injection parameter of at least one of the group of: delivered iodine concentration, injection timing, injection volume, injection speed, viscosity of iodine concentration, and injection real-time pressure curve. The term "real-time pressure curve" relates to real-time concentration of iodine injected, i.e. the pressure with which the concentration is injected.

In an example, the contrast of an observed vasculature is estimated by injecting contrast agent in a vasculature. This contrast agent replaces in part blood. The replacement level is characterized by the so-called mixing factor. In coronaries for example, a good practice is to obtain a backflow in the aorta, which means that virtually all the blood in vessels is replaced by contrast agent. In this case, absorption can be determined from the dilution and nature of the contrast agent, plus the geometry of the vessels' lumens (diameter and orientation with respect to X-ray projection direction). In another case, where contrast agent is mixed with the blood flow, the actual dilution after mixing depends on the mixing factor, which is related to the flow-rate ratio between blood and contrast agent.

In an example, for step 302 it is provided the step of acquiring the X-ray image of a vascular structure with an acquisition parameter setting that forms the current acquisition parameter setting.

In an optionally shown example, a step 312 is provided for applying the adaptive image contrast enhancement to the acquired X-ray image of the vascular structure and, as a result, a dynamically contrast enhanced X-ray image is generated.

In an example, in step 304, an adapted patient model is further provided based on the assessed patient built and the geometry parameter setting.

In a further, not shown example, an adapted vascular structure is further provided based on the assessed patient built, the generic vascular structure and the geometry parameter setting. By providing a specific vascular structure of the patient the assessed adaptive image contrast enhancement can be determined even more precisely.

In a not shown example, a further step is provided for determining an assessed scatter model at detector based on the assessed patient built and the current acquisition parameter. The scatter model at detector may involve characteristics of an applied anti-scatter grid. An anti-scatter grid is a device for limiting the amount of radiation scatter created in a radiographic exposure reaching the detector.

Fig. 4 shows another exemplary embodiment of a method 400. In step 402 a generic patient model including the targeted vascular structure is provided. In step 404, exposure parameters (aka Technique Factors), such as the kV value and the Cu thickness, are provided, which are usually predicted from a previous irradiation. They allow estimating the built of the patient. In a step 406, gantry geometrical settings and detector geometrical settings, such as magnification and format selection are provided. Optional, also a refined variant of the generic vessel model can be generated. Further, absorbers currently used (wedge filters, bowtie filters, neck filters, leg filters, ...) can be considered as another refinement "extension" to the patient model. In a next step 408, the amount of scatter expected at the detector entrance may be calculated by combining the elements of the steps 402 and 404. In a next step 410, the contrast of those vessels prior boosting may be estimated by combining the elements of the exposure parameters (such as kV), the generic patient model, the generic vessel models, the geometrical refinements and the scatter model. Based on this estimation, the Vessel Contrast Enhancement can be determined according to the ambition in a next step 412. In other words, dynamic contrast enhancement is provided.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for image contrast enhancement of an X-ray image of a vascular structure, the device comprising:
- an input unit (12); and
- a processing unit (14);
wherein the input unit is configured to provide a current acquisition parameter setting having at least one acquisition parameter; and to provide a generic vessel model;
wherein the processing unit is configured to assess a current patient build based on the current acquisition parameter setting; to assess at least one contrast parameter for at least a part of the main vessels of the generic vessel model based on the assessed current patient build and the current acquisition parameter setting; to determine an adaptive image contrast enhancement based on the at least one assessed contrast vessel parameter; and
wherein the adaptive image contrast enhancement is applied to an X-ray image of a vascular structure, which is acquired with the current acquisition parameter setting, in order to generate a contrast enhanced X-ray image.

2. Device according to claim 1, wherein an output unit (26) is provided;
wherein the processing unit is configured to apply the adaptive image contrast enhancement to the acquired X-ray image of the vascular structure and to generate a dynamically contrast enhanced X-ray image; and
wherein the output unit is configured to provide the contrast enhanced X-ray image; and
wherein, preferably, the output unit is a display unit.

3. An X-ray imaging system (20) comprising:
- an X-ray imaging arrangement (22); and
- a device (10) for image contrast enhancement of an X-ray image of a vascular structure according to one of the preceding claims;
wherein the X-ray imaging arrangement is configured to acquire the X-ray image of a vascular structure with the current acquisition parameter setting.

4. System according to claim 3, wherein the current acquisition parameter setting comprises:
i) an exposure parameter setting of at least one of the group of kV-value and filter; and/or
ii) a geometry parameter setting at least one of the group of gantry geometry parameter and detector geometry parameter.

5. System according to claim 3 or 4, wherein the current acquisition parameter setting comprises an absorber parameter setting at least one of the group of: wedge filters, bowtie filters, neck filter, leg filters.

6. A method (300) for image contrast enhancement of an X-ray image of a vascular structure, the method comprising the following steps:
a) providing (302) a current acquisition parameter setting having at least one acquisition parameter;
b) assessing (304) a current patient build based on the current acquisition parameter setting;
c) providing (306) a generic vessel model;
d) assessing (308) at least one contrast parameter for at least a part of the main vessels of the generic vessel model based on the assessed current patient build and the current acquisition parameter setting;
e) determining (310) an adaptive image contrast enhancement based on the at least one assessed contrast vessel parameter; wherein the adaptive image contrast enhancement is applied to an X-ray image of a vascular structure, which is acquired with the current acquisition parameter setting, in order to generate a contrast enhanced X-ray image.

7. Method according to claim 6, wherein a step a1) is provided, in which the X-ray image of the vascular structure is acquired with an acquisition parameter setting that forms the current acquisition parameter setting.

8. Method according to claim 6 or 7, wherein, following step e), the adaptive image contrast enhancement is applied (312) to the acquired X-ray image of the vascular structure and a dynamically contrast enhanced X-ray image is generated.

9. Method according to one of the claims 6 to 8, wherein in step b), an adapted patient model is further provided based on the assessed patient built and the geometry parameter setting.

10. Method according to one of the claims 6 to 9, wherein in step b), an adapted vascular structure is further provided based on the assessed patient built, the generic vascular structure and the geometry parameter setting.

11. Method according to one of the claims 6 to 10, wherein the method further comprises a step of:
c1) determining an assessed scatter model at detector based on the assessed patient built and the current acquisition parameter setting.

12. A computer program element for controlling an apparatus according to any one of claims 1 to 5, which, when being executed by a processing unit, is adapted to perform the method steps of one of the claims 6 to 11.

13. A computer readable medium having stored the program element of claim 12.
